# EUROPEAN PATENT SPECIFICATION

(11) **EP 1 929 017 B1**
(45) Date of publication and mention of the grant of the patent: **07.12.2011**
(21) Application number: 06793498.4
(22) Date of filing: 14.09.2006
(51) Int. Cl.: C12N 15/82

(54) **TRANSPLASTOMIC PLANTS EXPRESSING LUMEN-TARGETED PROTEIN**
TRANSPLASTOMISCHE PFLANZEN, DIE LUMEN-GEZIELTES PROTEIN ANGEBEN
PLANTES TRANSPLASTOMIQUES EXPRIMANT UNE PROTÉINE À DESTINATION DU LUMEN

(30) Priority: 16.09.2005 EP 05356163; 20.09.2005 US 718931 P
(43) Date of publication of application: 11.06.2008
(73) Proprietor: Bayer CropScience AG, 40789 Monheim (DE)
(72) Inventor: TISSOT-LECUELLE, Ghislaine, 73000 Chambéry (FR); CANARD, Hélène, F-69270 Fontaines sur Saône (FR); DUBALD, Manuel, F-69370 Saint Didier au Mont d'Or (FR)
(74) Representative: Guitton, Carole
(86) International application number: PCT/EP2006/066345
(87) International publication number: WO 2007/031547

(56) References cited:
- WO-A-01/04327
- WO-A-2004/053135
- US-A1- 2003 033 636
- US-A1- 2003 204 864
- DE BOER D ET AL: "PROTEIN TARGETING TOWARDS THE THYLAKOID LUMEN OF CHLOROPLASTS PROPER LOCALIZATION OF FUSION PROTEINS IS ONLY OBSERVED IN-VIVO" EMBO (EUROPEAN MOLECULAR BIOLOGY ORGANIZATION) JOURNAL, vol. 10, no. 10, 1991, pages 2765-2772, XP009059551 ISSN: 0261-4189
- HENRY RALPH ET AL: "Differences between lumen targeting domains of chloroplast transit peptides determine pathway specificity for thylakoid transport" JOURNAL OF BIOLOGICAL CHEMISTRY, vol. 269, no. 14, 1994, pages 10189-10192, XP002361478 ISSN: 0021-9258
- WESTERLUND ISABELLE ET AL: "LumenP: A neural network predictor for protein localization in the thylakoid lumen." PROTEIN SCIENCE, vol. 12, no. 10, October 2003 (2003-10), pages 2360-2366, XP002361479 ISSN: 0961-8368

### SEQUENCE LISTING

<110> Bayer CropScience SA
<120> Transplastomic plants expressing lumen-targeted protein
<130> BCS 05-4032
<160> 10
<170> PatentIn version 3.3
<210> 1
   <211> 138
   <212> DNA
   <213> Artificial
<220>
   <223> optimized nucleotide sequence LSP1
<220>
   <221> CDS
   <222> (1)..(138)
<400> 1
   atg tca aga ctt tct tta aaa act tct ggt gat gaa gaa aat tgg gtt       48
   Met Ser Arg Leu Ser Leu Lys Thr Ser Gly Asp Glu Glu Asn Trp Val
   1               5                   10                  15
   tca cga ttc aga tca aaa tct tta tca ttg gta ttt tct gga gtt tta       96
   Ser Arg Phe Arg Ser Lys Ser Leu Ser Leu Val Phe Ser Gly Val Leu
               20                  25                  30
   gct ctt ggt tta tct ctt tca gga gtt ggt ttt gca gat gca               138
   Ala Leu Gly Leu Ser Leu Ser Gly Val Gly Phe Ala Asp Ala
           35                  40                  45
<210> 2
   <211> 46
   <212> PRT
   <213> Artificial
<220>
   <223> Synthetic Construct
<400> 2
   Met Ser Arg Leu Ser Leu Lys Thr Ser Gly Asp Glu Glu Asn Trp Val
   1               5                   10                  15
   Ser Arg Phe Arg Ser Lys Ser Leu Ser Leu Val Phe Ser Gly Val Leu
               20                  25                  30
   Ala Leu Gly Leu Ser Leu Ser Gly Val Gly Phe Ala Asp Ala
           35                  40                  45
<210> 3
   <211> 126
   <212> DNA
   <213> Artificial
<220>
   <223> Optimized nucleotide sequence LSP2
<220>
   <221> CDS
   <222> (1)..(126)
<400> 3
   atg cga cca att aat caa gtt gta gct ttt tct gtt cct ata tca aga      48
   Met Arg Pro Ile Asn Gln Val Val Ala Phe Ser Val Pro Ile Ser Arg
   1               5                   10                  15
   aga gat gct agt ata att tta ctt tca tct att cca tta act tca ttt      96
   Arg Asp Ala Ser Ile Ile Leu Leu Ser Ser Ile Pro Leu Thr Ser Phe
               20                  25                  30
   ttc gta ctt act cct tca tct tca gaa gct                              126
   Phe Val Leu Thr Pro Ser Ser Ser Glu Ala
           35                  40
<210> 4
   <211> 42
   <212> PRT
   <213> Artificial
<220>
   <223> Synthetic Construct
<400> 4
   Met Arg Pro Ile Asn Gln Val Val Ala Phe Ser Val Pro Ile Ser Arg
   1               5                   10                  15
   Arg Asp Ala Ser Ile Ile Leu Leu Ser Ser Ile Pro Leu Thr Ser Phe
               20                  25                  30
   Phe Val Leu Thr Pro Ser Ser Ser Glu Ala
           35                  40
<210> 5
   <211> 174
   <212> DNA
   <213> Bos taurus
<220>
   <221> CDS
   <222> (1)..(174)
<400> 5
   cgg cct gac ttc tgc cta gag cct cca tat acg ggt ccc tgc aag gcc      48
   aga att atc aga tac ttc tac aac gcc aag gct ggg ctc tgc cag acc      96
   ttt gta tat ggc ggc tgc aga gct aaa aga aac aat ttc aag agc gca      144
   gag gac tgc atg agg acc tgt ggt ggt gct                              174
<210> 6
   <211> 58
   <212> PRT
   <213> Bos taurus
<400> 6
<210> 7
   <211> 26
   <212> DNA
   <213> Artificial
<220>
   <223> primer
<400> 7
   atgtcaccac aaacagagac taaagc 26
<210> 8
   <211> 26
   <212> DNA
   <213> Artificial
<220>
   <223> primer
<400> 8
   gttgatactt cggcgatcac cgcttc 26
<210> 9
   <211> 25
   <212> DNA
   <213> Artificial
<220>
   <223> primer
<400> 9
   tttgtagaaa actagtgtgc ttggg 25
<210> 10
   <211> 26
   <212> DNA
   <213> Artificial
<220>
   <223> primer
<400> 10
   atgtcaccac aaacagagac taaagc 26

## Description

The present invention relates to constructs and methods for the expression of recombinant proteins in the thylakoid lumen of transplastomic plant cells.

Plant plastids (chloroplasts, amyloplasts, etioplasts, chromoplasts, etc.) are derived from a common precursor known as a proplastid, and are responsible for the production of important compounds such as amino acids, complex carbohydrates, fatty acids and pigments. In general, plant cells contain 500-10,000 copies of a small 120-160 kilobase circular plastid genome containing single-copy and duplicated DNA segments. Thus, it is possible to engineer plant cells to contain up to 20,000 copies of a particular gene of interest which potentially can result in very high levels of foreign gene expression and an accumulation of recombinant proteins ranging up to 40% of total soluble cell proteins (De Cosa et al, 2001, Nat. Biotechnol.19, 71-74). Further, no gene silencing has been reported in chloroplast transgenic lines despite the accumulation of transcripts at a level 169 times higher than nuclear transgenic plants (Lee et al, 2003, Mol. Breeding, 11, 1-13). In addition, plastids of most plants are maternally inherited. Consequently, unlike heterologous genes expressed in the nucleus, heterologous genes expressed in plastids are not pollen disseminated. This particularity therefore greatly limits the risk of dispersion of the transgene in the environment, and its propagation to neighboring plants.

Chloroplasts are complex organelles in structural terms, comprising three distinct soluble phases. The chloroplast is bound by a double-membrane envelope, which encloses an intermembrane space. The major soluble phase is the stroma, which is the site of carbon fixation, amino acids synthesis and many other pathways. The dominant membrane is the extensive interconnecting thylakoid network, where light is captured and ATP synthesized. The thylakoid membrane encloses the third soluble phase, the thylakoid lumen, which houses a number of extrinsic photosynthetic proteins as well as many others (C. Robinson et al, 2001, Traffic 2:245-251).

Only a few chloroplast proteins are encoded and synthesized within the organelle. Most are encoded in the nucleus, synthesized as precursors in the cytosol, and post-translationally imported into one of the chloroplast subcompartments. This requires specific cellular sorting signals, which can be particularly sophisticated, due to the presence of the different membrane systems which can have to be crossed.

Two different secretion pathways for targeting proteins to the thylakoid lumen of chloroplast have been characterized. The first is the Sec-dependent pathway related to the SecYEG export mechanism in bacteria. The second is a pH-dependent mechanism, characterized by two conserved successive arginines in the transit peptide, the Tat (twin arginine translocase) pathway. Proteins targeted to the lumen via the Sec pathway are generally translocated in an unfolded state, whereas proteins imported *via* the Tat pathway can be translocated in a folded state. Proteins imported into the lumen by either pathway are processed by a thylakoid processing protease that removes the carboxyl-proximal portion of the transit peptide (C. Robinson et al, traffic 2001, 2:245-251).

The thylakoid lumen of chloroplasts is a plant cellular compartment which might be optimal for the accumulation of certain recombinant proteins due to its increasing oxidative stability, and its particular content in proteases (Z. Adams et al., TRENDS in Plant science, vol 7 N° 10, 2002). Despite this, it has rarely been considered for recombinant protein targeting and accumulation.

In the US patent US 6,512,162, the aprotinin coding sequence is fused with the *petA* gene in order to target the fused petA::aprotinin protein to the plant cell thylakoid membrane. *PetA* is a gene from the chloroplast genome encoding the cytochrome f (petA) protein, which has been reported as a polypeptide with a transmembrane arrangement in the chloroplast thylakoid membrane, with the N-terminal region in the intrathylakoid space, and a 15 amino acid C terminal sequence in the stroma (S.J. Rothstein et al, Proc. Natl. Acad. Sci. USA, Vol 82 pp 7955-7959, 1985). Therefore, the fused petA::aprotinin protein wherein the aprotinin coding sequence is linked to the 3' terminus of the coding sequence of cytochrome f (petA) should address the aprotinin in the stroma.

There is therefore still a need for methods and means which clearly and unambiguously address a peptide of interest in the thylakoid lumen of chloroplast.

The present invention provides for the first time nucleic acid sequences useful in targeting a recombinant protein encoded by a transgene integrated into the chloroplast genome to the thylakoid lumen of chloroplast, using lumen targeting signal sequence from nuclear-encoded proteins.

Such a strategy takes advantage of the high-level expression for transgenes integrated into the chloroplast genome in order to accumulate high amount of recombinant protein in a cell compartment having particular characteristics, especially in term of redox properties, proteases content and folding activities.

Additionally, it is possible with this strategy to produce recombinant proteins having a non-methionine N-terminus in plant chloroplasts.

### DESCRIPTION OF THE FIGURES

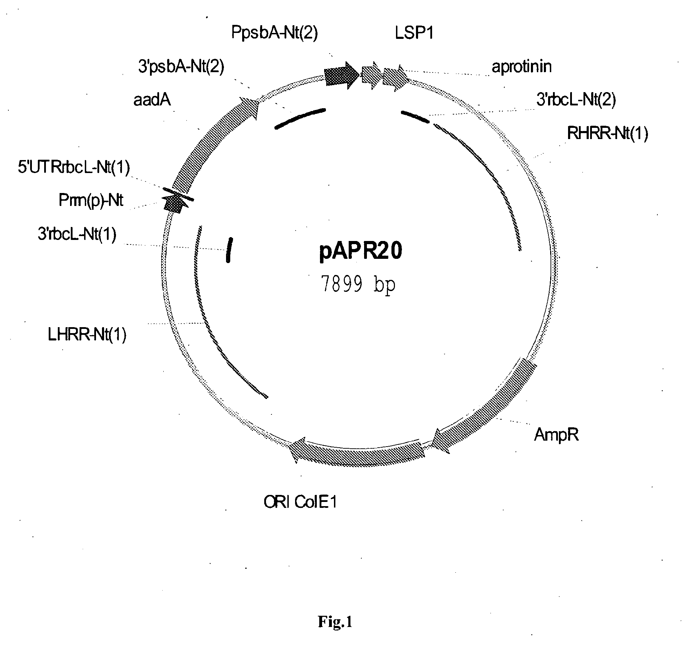

### Figure 1: map of plasmid pAPR20

The subject of the invention is a chimeric gene comprising, linked to one another in a functional fashion in the direction of transcription:
a) a promoter functional in a plant plastid
b) a lumen targeting domain of plastid transit peptide sequence, translationally fused with,
c) a heterologous nucleic acid sequence encoding a peptide,
d) optionally a terminator which is active in the plastids of plant cells, characterized in that said chimeric gene does not comprise the stromal targeting domain from said plastid transit peptide sequence and wherein expression for said chimeric gene is at least 0.1 % of the total soluble proteins.

Nuclear-encoded proteins targeted to the chloroplast thylakoid lumen compartment have a characteristic bipartite transit peptide, composed of a stromal targeting signal peptide and a lumen targeting signal peptide. The stromal targeting information is in the amino-proximal portion of the transit peptide. The lumen targeting signal peptide is in the carboxyl-proximal portion of the transit peptide, and contains all the information for targeting to the lumen. The lumen targeting signal peptides show strong similarities to bacterial signal peptides and can be divided into a charged N-terminal domain, a hydrophobic core and a more polar C-terminal domain that ends with short chain residues at the -3 and -1 position relative to the terminal cleavage site (von Heijne et al., Eur. J. Biochem. 80, 535-545, 1989).

Prediction of a lumen targeting signal peptide from a nuclear-encoded protein based on the amino acid sequence of the protein or on the nucleic acid sequence of the corresponding gene is well known to the skilled person.
As a non-limiting example, SignalP (Nielsen et al., Int. J. Neural Syst. 8:581-599, 1997) is a suitable tool to predict the bipartite transit peptides and lumen targeting signal peptides of lumenal proteins. Another way to proceed is to use the software TargetP (Emanuelsson et al, J Mol Biol 300:1005-1016, 2000) which allows for the large-scale prediction of the subcellular location of nuclear-encoded proteins and to research by a manual screening the TargetP-predicted chloroplast proteins for twin-arginine motifs (Kieselbach et al, Photosynthesis research, 78:249-264, 2003).

Recent research in proteomics of the higher plant chloroplast has achieved in the identification of numerous nuclear-encoded lumen proteins (Kieselbach et al. FEBS LETT 480:271-276, 2000; Peltier et al. Plant Cell 12:319-341, 2000; Bricker et al. Biochim. Biophys Acta 1503:350-356, 2001), the lumen targeting signal peptide of which can potentially be used in accordance with the present invention. About 80 proteins from *Arabidopsis,* as well as homologous proteins from spinach and garden pea, are reported by Kieselbach et al., Photosynthesis research, 78:249-264, 2003. In particular, table 2 of this publication, which is incorporated into the description herewith by reference, discloses 85 proteins from the chloroplast lumen, identified by their accession number. In addition, the recently published draft version of the rice genome (Goff et al, Science 296:92-100, 2002) is a suitable source for lumen targeting signal peptide which may be used in accordance with the present invention.

It is well known to the skilled person that normal translation in plastids initiates at methionine. Lumen targeting signal peptide from a nuclear-encoded protein may have a methionine as N-terminal amino acid or not.
In accordance with the invention, an ATG translation start codon, coding for a methionine, is fused in frame at the 5'end of the nucleic acid molecule encoding a lumen targeting signal peptide or substituted to the N-terminus amino acid when such lumen targeting signal peptide does not start by a methionine.

Nucleic acid molecules encoding a lumen targeting signal peptide may be isolated e.g. from genomic DNA or DNA libraries produced from plant origin. Alternatively, they may have been produced by means of recombinant DNA techniques (e.g. PCR) or by means of chemical synthesis. The identification and isolation of such nucleic acid molecules may take place by using the molecules according to the invention or parts of these molecules or, as the case may be, the reverse complement strands of these molecules, e.g. by hybridization according to standard methods (see e.g. Sambrook et al., 1989, Molecular Cloning, A Laboratory Manual, 2nd Edition, Cold Spring Harbor Laboratory Press, Cold Spring Harbor, N.Y.).
A methionine N-terminus lumen targeting signal peptide according to the invention can be obtained from a nuclear-encoded protein using techniques which are familiar to those skilled in the art, notably methods such as those described in Sambrook et al (1989, Molecular Cloning, a Laboratory Manual, Nolan C., ed., New Yyork: Cold Spring Harbor Laboratory Press).

Nuclear-encoded proteins can be targeted into the chloroplast thylakoid lumen compartment across the thylakoid membrane by two different secretion pathways. The first one is the Sec-dependent pathway, the second is the Tat pathway (Robinson et al, Plant Mol Biol 38,209-221, 1998; Cline et al, Annu. Rev. Cell dev. Biol. 12, 1-26, 1996). The inventors have shown that lumen targeting signal peptides coming from either a nuclear-encoded protein using the Sec-dependant pathway or either a nuclear-encoded protein using the Tat pathway are suitable in accordance with the present invention.

Thereby, the present invention relates to a chimeric gene as defined above, wherein the nucleic acid sequence encoding a lumen targeting signal peptide is from a nuclear-encoded protein using the Sec-dependent pathway, or using the Tat pathway, for the translocation across the thylakoid membrane.

In an embodiment of the present invention the nucleic acid sequence encoding a methionine N-terminus lumen targeting signal peptide is chosen from the group consisting of:
a) Nucleic acid molecule which encodes a peptide comprising the amino acid sequence given under SEQ ID N0 : 2 or 4;
b) Nucleic acid molecule which encodes a peptide, the amino acid sequence of which has an identity of at least 70%, at least 80%, at least 90%, 95% or 99% with the amino acid sequence given under SEQ ID N0:2 or 4;
c) Nucleic acid molecule, comprising the nucleotide sequence shown under SEQ ID N0 1 or 3;
d) Nucleic acid molecule, the nucleic acid sequence of which has an identity of at least 50%, at least 60%, at least 70%, 80% or 90% with the nucleic acid sequences described under a) or c);
e) Nucleic acid molecules, the nucleotide sequence of which deviates from the sequence of the nucleic acid molecules identified under a), b), c) or d) due to the degeneration of the genetic code; and
f) Nucleic acid molecules, which represent fragments, allelic variants and/or derivatives of the nucleic acid molecules identified under a), b), c), d) or e).

In accordance with the present invention, the term "identity" is to be understood to mean the number of amino acids/nucleotides corresponding with the amino acids/nucleotides of other protein/nucleic acid, expressed as a percentage. Identity is preferably determined by comparing the Seq. ID NO: 1, SEQ ID NO: 2, Seq. ID NO: 3 or SEQ ID NO: 4 with other protein/nucleic acid with the help of computer programs. If sequences that are compared with one another have different lengths, the identity is to be determined in such a way that the number of amino acids, which have the shorter sequence in common with the longer sequence, determines the percentage quotient of the identity. Preferably, identity is determined by means of the computer program ClustalW, which is well known and available to the public (Thompson et al., Nucleic Acids Research 22 (1994), 4673-4680). ClustalW is made publicly available by Julie Thompson (Thompson@EMBL-Heidelberg.DE) and Toby Gibson (Gibson@EMBL-Heidelberg.DE), European Molecular Biology Laboratory, Meyerhofstrasse 1, D 69117 Heidelberg, Germany. ClustalW can also be downloaded from different Internet sites, including the IGBMC (Institut de Génétique et de Biologie Moléculaire et Cellulaire, B.P.163, 67404 Illkirch Cedex, France; ftp:Hftp-igbmc.u-strasbg.fr/pub/) and the EBI (ftp:Hftp.ebi.ac.uk/pub/software/) as well as from all mirrored Internet sites of the EBI (European Bioinformatics Institute, Wellcome Trust Genome Campus, Hinxton, Cambridge CB10 1SD, UK).
Preferably, Version 1.8 of the ClustalW computer program is used to determine the identity between proteins according to the invention and other proteins. In doing so, the following parameters must be set: KTUPLE=I, TOPDIAG=5, WINDOW=5, PAIRGAP=3, GAPOPEN=10, GAPEXTEND=0.05, GAPDIST=8, MAXDIV=40, MATRIX=GONNET, ENDGAPS(OFF), NOPGAP, NOHGAP.

Preferably, Version 1.8 of the ClustalW computer program is used to determine the identity between the nucleotide sequence of the nucleic acid molecules according to the invention, for example, and the nucleotide sequence of other nucleic acid molecules. In doing so, the following parameters must be set:
KTUPLE=2, TOPDIAGS=4, PAIRGAP=5, DNAMATRIX:IUB, GAPOPEN=10, GAPEXT=5, MAXDIV=40, TRANSITIONS: unweighted.

In accordance with the present invention, the terms "linked to one another in a functional fashion" or "operably linked" means that the specified elements of the component chimeric gene are linked to one another in such a way that they function as an unit to allow expression of the coding sequence. By way of example, a promoter is said to be linked to a coding sequence in a functional fashion if it is capable of promoting the expression of said coding sequence.
In accordance with the present invention, the terms ""linked to one another in a functional fashion" cover the case of a polycistronic arrangement wherein the promoter is not directly linked to the coding sequence.

A chimeric gene according to the invention can be assembled from the various components using techniques which are familiar to those skilled in the art, notably methods such as those described in Sambrook et al (1989, Molecular Cloning, a Laboratory Manual, Nolan C., ed., New york: Cold Spring Harbor Laboratory Press). Exactly which regulatory elements are to be included in the chimeric gene would depend on the plant and the type of plastid in which they are to work: those skilled in the art are able to select which regulatory elements are going to work and can improve the production of protein into a given plant. As an example, the Shine-Dalgamo (SD) consensus sequence GGAGG can be placed upstream of the gene. Alternatively or in addition, a 5' untranslated region (UTR) can be inserted between the promoter and the gene (Staub J.M. and Maliga P., 1993, EMBO J. 12, 601-606). Those skilled in the art are aware than the use of 5' untranslated region (5'UTR) and 3' untranslated region (3'UTR) regulatory signals are generally necessary for higher levels of transgene expression in plastids (De Cosa B., Moar W., Lee S.B., Miller M. and Daniell H., 2001, Nat. Biotechnol. 19, 71-74). Possible 5'UTR and 3'UTR are well known by those skilled in the art. As an example, the promoter of the *psbA* gene, nucleotide 1596 to 1819 from Genbank Z00044, includes the endogenous 5'UTR. The promoter of the 16S ribosomal operon *Prrn* can be associated with the ribosome binding site region of the *rbcL* gene (5'UTR rbcL).

Among the promoters functional in plastids of plant cells, by way of example, special mention can be made of the *psbA* gene which encodes the D 1 polypeptide of PSII (Staub et al. 1993 EMBO Journal 12(2):601-606), and the constitutive *Prrn* promoter which regulates the ribosomal RNA operon (Staub et al. 1992 Plant Cell 4:39-45). As a general rule, any promoter resulting from a plastomic plant gene or a bacterial gene could work, and those skilled in the art will know which of the available promoters to select in order to obtain the desired mode of expression (constitutive or inducible).

A well-suited promoter for the current invention is the *Prrn* promoter of tobacco which is associated with part of the 5' untranslated sequence of the *rbcL* gene providing a ribosome-binding site (Svab et al., 1993, Proc. Natl. Acad. Sci. 90:913-917).

Another well-suited promoter is the light-dependent promoter of the *psbA* gene which encodes the D1 polypeptide of PSII (Staub J.M. and Maliga P., 1993, EMBO J. 12, 601-606).

Among the terminators which are active in plant cell plastids, by way of example, special mention could be made of the terminators of the *psbA* gene, the *rbcL* gene (which codes for the large sub-unit of RuBisCO), and the *rps16* gene (which codes for a tobacco ribosomal protein) (Shinozaki et al., 1986, EMBO J. 5:2043-2049; Staub J.M. and Maliga P., 1993, EMBO J. 12, 601-606).

In accordance with the present invention, the term "translationally fused with" shall mean a fusion of nucleic acid sequences in such a way that they represent a single open reading frame, which upon transcription leads to the production of a single messenger RNA encoding a single polypeptide, when translated.

In accordance with the present invention, the nucleic acid sequence encoding a methionine N-terminus lumen targeting signal peptide from a nuclear-encoded protein is translationally fused with a heterologous nucleic acid sequence, which means that this second nucleic acid sequence is not naturally fused with the first nucleic acid sequence encoding a lumen targeting signal peptide.

In an embodiment of the present invention, the heterologous nucleic acid sequence encoding a peptide which is fused to the nucleic acid sequence encoding a lumen targeting signal peptide, is derived from an eukaryotic organism.

In another embodiment of the invention, the nucleic acid sequence encoding a methionine N-terminus lumen targeting signal peptide and/or the heterologous nucleic acid molecule are designed in order to optimize chloroplast expression, based on the chloroplast codon usage of *Nicotiana tabacum.* The chloroplast codon usage of *Nicotiana tabacum* is available on www. Kazusa.or.jp/codon, and the distribution of the codons is randomly attributed to each amino acid residue over the entire coding sequence according to the frequency in the chloroplast codon usage table (Nakamura et al., 2000, Nucl. Acids Res. 28, 292).

In yet another embodiment of the invention, the heterologous nucleic acid sequence encoding a peptide encodes a peptide having a non-methionine N-terminus.

Heterologous nucleic acid molecule encoding a peptide may be isolated e.g. from genomic DNA or DNA libraries produced from eukaryotic or other origin. Alternatively, they may have been produced by means of recombinant DNA techniques (e.g. PCR) or by means of chemical synthesis. The identification and isolation of such nucleic acid molecules may take place by using the molecules according to the invention or parts of these molecules or, as the case may be, the reverse complement strands of these molecules, e.g. by hybridization according to standard methods (see e.g. Sambrook et al., 1989, Molecular Cloning, A Laboratory Manual, 2nd Edition, Cold Spring Harbor Laboratory Press, Cold Spring Harbor, N.Y.).

In yet another embodiment of the present invention, the heterologous nucleic acid sequence encoding a peptide encodes the aprotinin protein. Aprotinin is a protease inhibitor which can be extracted from bovine organs or tissues, such as pancreas, lungs, or liver. Aprotinin is known to inhibit various serine proteases, including trypsin, chymotrypsin, plasmin and kallikrein, and is used therapeutically in the treatment of the myocardial infarction, shock syndrome, hyperfibrinolytic and acute pancreatitis, and in order to reduce blood loss in connection with cardiac surgery (Bidstrup et al, 1989, Cardiovasc Surg. 44:640-645). The nucleic acid and amino acid sequences of aprotinin can be found in the Swiss-Prot/TrEMBL database (collaboration between the Swiss Institute of Bioinformatics and the EMBL outstation - the European Bioinformatics Institute; *httD:*//*us. expasy.org*/*sprot)* under the Accession Number P00974.

In accordance with the present invention, the aprotinin protein is identified under SEQ ID N0 : 6. A chimeric gene according to the invention wherein the heterologous nucleic acid sequence encodes a peptide containing fragments of the aprotinin protein exhibiting biological activity comparable to the aprotinin protein , or encodes a peptide , the amino acid sequence of which has an identity to at least 70%, at least 80%, at least 90%, 95% or 99% to SEQ ID N0 6 is a further embodiment of the invention.

In an other embodiment of the invention, the heterologous nucleic acid sequence has a nucleic acid sequence with an identity of at least 50 %, at least 60%, at least 70%, or at least 75 % to the sequence SEQ ID N0 : 5.

The nucleic acid molecules or the polypeptides, which are homologous to other molecule and constitute derivatives of these molecules, are generally variations of these molecules, which constitute modifications, which execute the same biological function. For this purpose, modifications can occur on amino-acid residues not involved in the enzyme activity.

In the context of the invention, the biological function of the nucleic acid molecules or of the polypeptides coded by them can be assessed by the ability for the polypeptides or for the polypeptides coded by the nucleic acid molecules to inhibit a serine protease, using for example an in-vitro test such as the Na-benzoyl-D,L-arginine-p-nitroanilide hydrochloride assay (Lavens et al, 1993, J. Immunol. Methods, 166(1), 93-102; Sigma-Aldrich, product N° B 4875, assay according to the manufacturer's manual). The variations, for example mutations, may have occurred in a natural manner or have been introduced by mutagenesis. The variations can also be synthetically manufactured sequences. The allelic variants can be both naturally occurring variants and also synthetically manufactured variants or variants produced by recombinant DNA techniques. Nucleic acid molecules, which deviate from nucleic acid molecules according to the invention due to degeneration of the genetic code, constitute a special form of derivatives.

Chimeric genes including a heterologous nucleic acid molecule that encodes aprotinin and the sequence of which differs from the nucleotide sequence of the native molecule due to the degeneracy of the genetic code are also the subject-matter of the invention.

Chimeric genes including a heterologous nucleic acid molecule that encodes variant aprotinin with alteration of the enzyme activity are also the subject-matter of the invention.

The invention also relates to a vector designated for the transformation of plant plastids, characterized in that it contains at least two sequences that are homologous to sequences in the plastome of the plant to be transformed, said homologous sequences flanking at least one chimeric gene according to the invention.

These sequences - one upstream (LHRR) and the other downstream (RHRR) of the component chimeric gene(s) - permit double homologous recombination within an intergenic region of the plastome, comprising the contiguous region LHRR and RHRR.

The two homologous recombination sequences according to the invention may be contiguous so that the chimeric gene is inserted at a non-coding (intergenic) sequence of the plastome. In a particular embodiment, this sequence is part of the operon of the plastid ribosomal RNA. In another particular embodiment, the non-coding sequence includes the 3' end of the *rbcl* gene (which codes for the large subunit of ruBisCO), with the other homologous sequence including the 5' end of the *accD* gene (which codes for one of the subunits of acetyl-CoA carboxylase). And more particularly still, the LHRR fragment corresponds to nucleotides 57764 to 59291 of the tobacco plastome (Shinozaki et al., 1986 - Genbank Z00044). The RHRR fragment corresponds to nucleotides 59299 to 60536 of the tobacco plastome.

To obtain plastid transformation, the transforming DNA must cross the cell wall, the plasma membrane and the double membrane of the organelle before reaching the stroma. In this respect, the most commonly used technique for transforming the plastid genome is that of particle bombardment (Svab and Maliga, 1993, Proc. Natl. Acad. Sci. USA, Feb 1, 90(3):913-917).
Plastid transfection using high velocity microprojectiles was first performed in the single-celled alga *Chlamydomonas reinhardtii* (Boynton et al., 1988). Currently, in higher plants, stable transformation of plastids is commonly carried out in tobacco, Nicotiana tabacum (Svab and Maliga, 1990, Proc. Natl. Acad. Sci. USA 87, 8526-8530; Svab and Maliga, 1993, Proc. Natl. Acad. Sci. USA, Feb 1, 90(3):913-917).

Transformation of plastids from rice (Khan M.S. and Maliga, 1999, Nat. Biotechnol. 17, 910-915), from *Arabidopsis thaliana* (Sikdar et al., 1998, Plant Cell Reports 18:20-24), from potato (Sidorov et al, 1999, Plant J. 19(2):209-216), from *Brassica napus* (Chaudhuri et al., 1999, WO 00/39313) and from tomato (Ruf et al., 2001, Nat. Biotechnol. 19, 870-875) have been reported. Fertile transplastomic plants have been obtained for tobacco, tomato, potato and soybean (WO 04/053133). Recently, transformation of duckweed plastids has been reported (WO 05/005643).

Selective marker may be used to select for transformed plastids and cells, i.e. those that have incorporated the chimeric gene(s) into their plastome (i.e. transplastomic cells), and it also makes it possible to obtain fertile, homoplasmic transplastomic plants. The term "homoplasmic" means that all the cells contain the same kind of plastome and only that plastome. Transplastomic plants are homoplasmic when all their cells contain only copy of the transformed plastome.

Among the genes that can be used as selective markers, by way of example, special mention can be made of two chimeric genes, namely the *aadA* gene which codes for an aminoglycoside 3"-adenyltransferase that confers resistance to spectinomycin and streptomycine (Svab et al., 1993, Proc. Natl. Acad. Sci. 90:913-917), and the *neo* gene which codes for a neomycin phosphotransferase (Carrer et al., 1993, Mol. Gen. Genet. 241:49-56) that confers resistance to kanamycin. Other suitable candidate selective markers include genes that confer resistance to betain aldehyde such as the gene that codes for betain aldehyde dehydrogenase (Daniell et al., 2001, Curr. Genet. 39:109-116), and also genes that confer herbicide tolerance such as the *bar* gene (White et al., 1990, Nucleic Acid Res. 18(4):1062) which confers resistance to bialaphos, and the EPSPS gene (US 5 188 642) which confers resistance to glyphosate. Alternatively, reporter genes can be used, i.e. genes that codes for readily identified enzymes such as GUS (β-glucuronidase) (Staub J.M. and Maliga P., 1993, EMBO J. 12, 601-606) or the green fluorescent protein (GFP, Sidorov et al., 1999, Plant J. 19(2):209-216), genes coding for pigments, or for enzymes that regulate pigment production. Such genes are described in Patent Applications WO 91/02071, WO 95/06128, WO 96/38567, WO 97/04130 and WO 01/64023.

The gene coding for the selective marker may be the *aadA* gene which codes for an aminoglycoside 3"-adenyltransferase that confers resistance to spectinomycin and streptomycine (Svab et al., 1993, Proc. Natl. Acad. Sci. 90:913-917).

The present invention therefore also relates to transplastomic plant cells, or transplastomic plants and/or progeny thereof, having integrated into their plastome a nucleic acid molecule comprising linked to one another in a functional fashion in the direction of transcription a promoter sequence which is active in plastids, a nucleic acid sequence encoding a methionine N-terminus lumen targeting signal peptide from a nuclear-encoded protein translationally fused with a heterologous nucleic acid sequence encoding a peptide, and optionally a terminator which is active in the plastids of plant cells.

In an embodiment of the present invention, the heterologous nucleic acid sequence encoding a peptide which is fused to the nucleic acid sequence encoding a lumen targeting signal peptide, is derived from an eukaryotic organism.

The present invention also relates to a transplastomic plant and/or progeny which is a Lemnaceae, a plant from the genus *Nicotiana*, a potato plant, a tomato plant, a soybean plant, or an algae.

In a particular embodiment, the transplastomic plant of the invention is a tobacco plant.

In a further embodiment, the present invention relates to harvestable plant parts of plants according to the invention, such as leaves, wherein these harvestable parts contain plant cells according to the invention.

The present invention also relates to a method for the manufacture of transplastomic plants according to the invention wherein
a) a plant cell is transformed with at least one chimeric gene which comprises, linked to one another in a functional fashion in the direction of transcription, a promoter sequence which is active in plastids, a lumen targeting domain of plastid transit peptide sequence, translationally fused with a heterologous nucleic acid sequence encoding a peptide, and optionally a terminator which is active in the plastids of plant cells, wherein said chimeric gene does not comprise the stromal targeting domain from said plastid transit peptide sequence and wherein expression for said chimeric gene is at least 0.1 % of the total soluble proteins;
b) a plant is regenerated from a plant cell obtained in step a) and
c) if necessary, further plants are produced from the plants obtained in step b).

The plant cell obtained in step a) may be regenerated to whole plants according to methods known to the skilled person, as for example using the methods described in "Plant Cell Culture Protocols" 1999, edited by R.D. Hall, Humana Press, ISBN 0-89603-549-2.

The production of further plants according to Step (c) of the method according to the invention can be carried out, for example, by vegetative propagation (for example using cuttings, tubers or by means of callus culture and regeneration of whole plants) or by sexual propagation. Here, sexual propagation preferably takes place under controlled conditions, i.e. selected plants with particular characteristics are crossed and propagated with one another.

The present invention further relates to a method for producing a protein of interest in a plant cell comprising the step of transforming a plastid with a chimeric gene according to the invention, and growing a plant cell comprising said transformed plastid under suitable conditions for the expression of the chimeric gene and for the subsequent cleavage of the signal peptide.

The present invention further relates to a method for producing a non-methionine N-terminus peptide in a plant plastid comprising the step of:
a) transforming a plastid with a chimeric gene which comprises, linked to one another in a functional fashion in the direction of transcription, a promoter sequence which is active in plastids, a lumen targeting domain of plastid transit peptide sequence translationally fused with a heterologous nucleic acid sequence encoding a peptide, and optionally a terminator which is active in the plastids of plant cells, wherein the heterologous nucleic acid sequence encodes a non-methionine N-terminus peptide, wherein said chimeric gene does not comprise the stromal targeting domain from said plastid transit peptide sequence and wherein expression for said chimeric gene is at least 0.1% of the total soluble proteins;
b) growing a plant cell comprising said transformed plastid under suitable conditions for the expression of the chimeric gene and for the subsequent cleavage of the signal peptide.

The present invention further relates to the above-described method for producing a non-methionine N-terminus peptide in a plant plastid, wherein the non-methionine N-terminus protein is the aprotinin.

The present invention further relates to a method for producing a peptide of interest comprising the step of extracting the peptide of interest from a transplastomic plant cell according to the invention, or from a transplastomic plant and/or progeny thereof according to the invention, or from harvestable parts of a transplastomic plant according to the invention.

Preferably, such a method also comprises the step of harvesting the cultivated plants and/or parts of such plants such as leaves before extracting peptide of interest. Most preferably, it further comprises the step of cultivating the plants of the invention before harvesting.

The present invention further relates to the above-described method for producing a peptide of interest wherein the peptide of interest is the aprotinin.

Methods for the extraction of the aprotinin are known to the skilled person. A number of methods are available to isolate aprotinin, including generally precipitation methods, and/or chromatographic steps using DEAE- cellulose or affinity techniques. Examples of such methods are described in the US patent US 5,164,482 and in J.D. Altman et al, 1991, Protein Eng, 4(5):593-600.

The invention is specifically illustrated by the following examples which are not in any way limitating.

### Example 1 : Nucleic acid sequence encoding a methionine-N-terminus lumen targeting signal peptide using the Sec-dependant pathway for the translocation across the thylakoid membrane

The *Arabidopsis thaliana* AT5g52970 gene of unknown function which encodes a protein secreted to the lumen via the Sec-dependant pathway has been selected among lumenal proteins identified by Kieselbach et al, 2003, Photosynthesis research, 78:249-264. This gene has been chosen among the lumenal proteins which have a positive charge (arginine or lysine) at the amino-terminus after cleavage of the signal peptide. Such a pre-selection has been done in order to improve the probability of a further exact processing of the fusion protein and removal of the aprotinin, the amino acid sequence of which starts with arginine. No pre-selection, or pre-selection based on other criteria, may be made depending on the heterologous nucleic acid sequence which will be translationally fused with the nucleic acid sequence encoding a methionine N-terminus lumen targeting signal peptide.

The bipartite transit peptide of the protein encoding by AT5g52970 was predicted using SignalP software (Nielsen et al., Int. J. Neural Syst. 8:581-599, 1997). The predicted amino-terminus sequence of this bipartite transit peptide allowing the import into the chloroplast was deleted and a methionine was added as translation start site, leading to the LSP1 sequence identified under SEQ ID N0 2.

### Example 2 : Nucleic acid sequence encoding a methionine-N-terminus lumen targeting signal peptide using the Tat pathway for the translocation across the thylakoid membrane

The *Arabidopsis thaliana* AT1g20810 gene encoding an immunophilin-like protein secreted to the lumen via the Tat pathway has been selected among lumenal proteins identified by Kieselbach et al, 2003, Photosynthesis research, 78:249-264. This gene has been chosen among the lumenal proteins which have a positive charge (arginine or lysine) at the amino-terminus after cleavage of the signal peptide. Such a pre-selection has been done in order to improve the probability of a further exact processing of the fusion protein and removal of the aprotinin, the amino acid sequence of which starts with arginine. No pre-selection, or pre-selection based on other criteria, may be made depending on the heterologous nucleic acid sequence which will be translationally fused with the nucleic acid sequence encoding a methionine N-terminus lumen targeting signal peptide.

The bipartite transit peptide of the protein encoding by AT1g20810 was predicted using SignalP software (Nielsen et al., Int. J. Neural Syst. 8:581-599, 1997). The predicted amino-terminus sequence of this bipartite transit peptide allowing the import into the chloroplast was deleted and a methionine was added as translation start site, leading to the LSP2 sequence identified under SEQ ID N0 : 4.

### Example 3: Coding sequences of LSPs/aprotinin fusion proteins

Synthetic sequences encoding LSP or LSP2 fused at the amino-terminus of aprotinin (named LSP1::aprotinin and LSP2::aprotinin respectively) were designed in order to optimize chloroplast expression, based on the tobacco plastid codon usage (www.kazusa.or.jp/codon). The distribution of the codons was randomly attributed to each amino-acid residue according to their frequency in the chloroplast codon usage table.

### Example 4: Chloroplast transformation vectors pAPR20 and pAPR21

The LSP1::aprotinin and LSP2::aprotinin coding sequences were introduced in a chloroplast transformation vector derived from pBluescript plasmid (Stratagene), giving vectors pAPR20 and pAPR21 respectively. pAPR20 is shown in Figure 1. pAPR21 is identical to pAPR20, except that LSP2 take the place of LSP1.
These vectors contain regions from the tobacco plastome, LHRR-Nt(1) and RHRR-Nt(1), allowing the targeted integration of the transgenes between the *rbcL* and *accD* genes. The LHRR fragment corresponds to nucleotides 57764 to 59291 of the tobacco plastome (Shinozaki et al., 1986 - Genbank Z00044). The RHRR fragment corresponds to nucleotides 59299 to 60536 of the tobacco plastome.
The chimeric selectable marker gene *aadA* encodes the resistance to spectinomycine as described by Svab and Maliga (1993). Its expression is placed under the control of the promoter of the 16S ribosomal operon (Prrn(p)-Nt: nucleotides 102561 to 102677 from Genbank Z00044), followed by the ribosome binding site of the rbcL gene (5'UTRrbcL-Nt: nucleotides 57569 to 57584 from Genbank Z00044). The transcription terminator, 3'psbA-Nt(2), comes from the *psbA* gene (nucleotides 146 to 533 from Genbank Z00044 in reverse orientation).
The LSPs::aprotinin fusion coding sequences are placed under the control of the promoter of the *psbA* gene (PpsbA-Nt(2): nucleotides 1596 to 1819 from Genbank Z00044 in reverse orientation). The transcription terminator, 3'rbcL-Nt(2), comes from the rbcL gene (nucleotides 59036 to 59246 from Genbank Z00044).
The remaining elements from the vector derive from the pBluescript vector.

### Example 5: Chloroplast transformation

*Nicotiana tabacum* (var. PBD6) plants were cultivated in sterile conditions on MS medium (Murashige and Skoog, 1962) supplemented with sucrose (3%) and phytagar (7 g/l). The abaxial sides of leaves measuring 3 to 5 centimeters were bombarded using a particle gun built in the laboratory according to the model described by Finer et al (1992). The DNA of vector pAPR23 (5 microgram per bombardment) was adsorbed onto gold particles in the presence of CaC12 (0.8 to 1.0 M) and spermidine (14 to 16 mM). The treated leaves were placed for 2 days on the same MS medium supplemented with naphtalene acetic acid (ANA 0.05 mg/l) and 6-benzylaminopurine (BAP 2 mg/l). The leaves were then cut into squares of in average 5 mm length and cultivated for selection of transplastomic events on the previous hormone-containing medium supplemented with 500 mg/l of spectinomycine hydrochloride. Leaf pieces were subcultured on fresh selection medium every 10 days. After 4 to 6 weeks, green calli or plantlets appearing on the bleached explants were isolated and transferred to MS medium (3% sucrose and 7 g/l phytagar) supplemented with 500 mg/l spectinomycine hydrochloride, without hormones. The regenerated shoots, once rooted, were then transferred to the greenhouse.

### Example 6: PCR analysis of selected lines

The first events generated with pAPR20 and pAPR21, selected on spectinomycine, were analyzed by PCR in order to check the presence of the transgenes at the expected location in the tobacco chloroplast genome. Two different couples of primers (1) and (2) were used for this analysis.
The first couple (1) allows the confirmation of the integration at the expected site with one primer landing in the *rbcL* gene before the LHRR fragment present in the transforming vector pAPR20 or pAPR21 (orbcL52F: 5'-atgtcaccacaaacagagactaaagc-3') and the second primer landing at the beginning of the AADA coding region, in reverse orientation (aadAlOR: 5'-gttgatacttcggcgatcaccgcttc-3'). The observation of an amplification product of approximately 1,8 kb will confirm the integration.
The second couple (2) allows the amplification of a fragment encompassing the LSPs::aprotinin fusion with one primer landing at the end of the *psbA* promoter (psbA230F: 5'-tttgtagaaaactagtgtgcttggg-3') and the second primer landing in the terminator 3'rbcL in reverse orientation (5'-atgtcaccacaaacagagactaaagc-3'). The observation of an amplification product of approximately 550 bp will confirm the presence of the LSPs::aprotinin expression cassettes in the tobacco chloroplast genome. The amplification consisted in 30 cycles (94°C for 45 seconds - 54°C for 60 seconds - 72°C for 120 seconds).
The PCR reactions for the 3 selected events (APR20-1-2, APR21-19-1, and APR21-19-2) are positive for each of the 2 couples of primers, showing that they are transplastomic and have integrated the expression cassettes of pAPR20 and pAPR21 as expected.

### Example 7: Western blot analysis of aprotinin

Expression of aprotinin was analyzed by SDS-PAGE under denaturing conditions followed by Western blot. Total soluble proteins were extracted from leaf material ground in liquid nitrogen of transgenic events using as extraction buffer Tris-HCl 25mM, NaCl 100mM, Triton X100 0,5%, glycerol 10%, pH8. Twenty micrograms of soluble proteins from each extract, quantified by a Bradford assay, were then separated by SDS-PAGE (12% acrylamide) and transferred on a PVDF membrane. A standard amount of aprotinin (200 ng; Sigma), as well as an extract from wild-type tobacco were also included in the experiment. After transfer, the membrane was blocked with blocking reagent according to the instructions of the manufacturer (Roche), and then incubated overnight at 4°C with a mouse monoclonal antibody directed against aprotinin. After washing, the membrane was incubated with a second monoclonal antibody directed against mouse immunoglobulins and coupled to alkaline phosphatase (Sigma A3562). Revelation of the immune complex was performed using a kit from Biorad (Immun-Star) and the generated chimioluminescence recorded on film.
The result of this experiment clearly shows that for both type of constructs (pAPR20 and pAPR21) the LSP::aprotinin fusion protein is processed and cleaved, since a unique band is visible on the western blot, which migrates at exactly the same position as genuine standard aprotinin. The selected signal peptides addressing aprotinin to the lumen through either the Sec (pAPR20 lines) or the Tat (pAPR21 lines) pathway are therefore cleaved with a high efficiency.
The level of expression is estimated at around 0.1-0.2% of total soluble proteins for pAPR21 lines and at around 0.5% for pAPR20 lines.

### Example 8 : Electrospray LC/MS analysis of recombinant aprotinin

Recombinant aprotinin was extracted in PBS buffer from transgenic leaves of events generated with pAPR20 and pAPR21 vectors and immunopurified using monoclonal antibodies. The isolated proteins were ziptiped to remove the salts and analyzed on a Ionics EP-10+ LC/MS/MS instrument.
The LC/MS electrospray chromatogram of the samples corresponding to pAPR20 and pAPR21 respectively has been compared to the LC/MS electrospray chromatogram of the aprotinin standard.
The LC/MS electrospray chromatogram of the samples corresponding to pAPR20 shows a peak at a retention time of 3.31 minutes. The average molecular weight obtained from the multiply charged ions is 6532 ± 2 Daltons. The difference in mass compared to the standard aprotinin is 16 daltons or the mass of oxygen. The isolated aprotinin appears to be oxidized. The LC/MS electrospray chromatogram of the sample corresponding to pAPR21 shows a peak at a retention time of 3.36 minutes. The average molecular weight obtained from the multiply charged ions is 6516 ± 1 Dalton. The mass spectrum is just like the standard aprotinin.

These experiments clearly show that a cleavage occurs *in planta* at the expected site between the signal peptide and the mature aprotinin, starting with arginine at the N-terminus, and that it corresponds to genuine aprotinin at the aminoacid level. The supplementary mass of 16 in the sample pAPR20 might correspond to the oxidation of one residue, possibly the unique encoded Methionine.

### Example 9: Recombinant aprotinin binds to trypsin

In order to check if recombinant aprotinin produced in transgenic chloroplasts is in an active conformation, extracts of leaf material from lines generated with vectors pAPR20 and pAPR21 were prepared in PBS buffer, and samples corresponding to 20 micrograms of total soluble protein were incubated for 30 minutes at 37°C with various amounts of trypsin (0, 28, 112, 450, or 1800 nanograms). Afterwards, samples were separated by SDS-PAGE under non-reducing conditions, and a western analysis performed using monoclonal antibodies directed against aprotinin. This analysis shows for both type of extracts the apparition of a higher molecular weight band as soon as trypsin is added to the samples. This new band corresponds to the complexation of aprotinin to trypsin. When enough trypsin is added, no free aprotinin remains, only the complex is detected.
This experiment shows that the totality of recombinant aprotinin produced in plastid transformants with vectors pAPR20 and pAPR21 is in an active conformation able to bind to the trypsin protease.

## Claims

1. A chimeric gene comprising, linked to one another in a functional fashion in the direction of transcription:
a) a promoter functional in a plant plastid,
b) a lumen targeting domain of plastid transit peptide sequence, translationally fused with
c) a heterologous nucleic acid sequence encoding a peptide,
d) optionally a terminator which is active in the plastids of plant cells,
**characterized in that** said chimeric gene does not comprise the stromal targeting domain from said plastid transit peptide sequence and wherein expression for said chimeric gene is at least 0.1 % of the total soluble proteins.

2. A chimeric gene according to claim 1, wherein the nucleic acid sequence encoding a lumen targeting signal peptide is from a nuclear-encoded protein using the Sec-dependent pathway or the Tat pathway for the translocation across the thylakoid membrane.

3. A chimeric gene according to any of claim 1 to 2, wherein the heterologous nucleic acid sequence encoding a peptide encodes the aprotinin protein.

4. A vector designated for the transformation of plant plastids, **characterized in that** it contains at least two sequences that are homologous to sequences in the plastome of the plant to be transformed, said homologous sequences flanking at least one chimeric gene according to any of claims 1 to 3.

5. A transplastomic plant cell, **characterized in that** it contains at least one chimeric gene according to any of claim 1 to 3.

6. A transplastomic plant and/or progeny thereof comprising a transplastomic plant cell according to claim 5.

7. A transplastomic plant and/or progeny thereof according to claim 6, which is an algae, a Lemnaceae , a plant from the genus *Nicotiana,* a potato, tomato or soybean plant.

8. Harvestable parts of a plant according to anyone of claim 6 to 7, comprising plant cells according to claim 5.

9. A method for producing a protein of interest in a plant cell comprising the following steps:
a) transforming a plastid with a chimeric gene according to any of claim 1 to 2;
b) growing a plant cell comprising said transformed plastid under suitable conditions for the expression of the chimeric gene and for the subsequent cleavage of the signal peptide.

10. A method for producing a non-methionine N-terminus peptide in a plant plastid, wherein said method comprises
a) transforming a plastid with a chimeric gene according to any of claim 1 to 2, wherein the heterologous nucleic acid sequence encodes a non-methionine N-terminus peptide
b) growing a plant cell comprising said transformed plastid under suitable conditions for the expression of the chimeric gene and for the subsequent cleavage of the signal peptide.

11. A method according to claim 10 wherein the non-methionine N-terminus protein is the aprotinin.

12. A method for producing a peptide of interest comprising the step of extracting the peptide of interest from a transplastomic plant cell according to claim 5, or from a transplastomic plant and/or progeny thereof according to anyone of claim 6 to 7, or from harvestable parts of a transplastomic plant according to claim 8.

13. A method according to claim 12 wherein the peptide of interest is the aprotinin.

## Patentansprüche

1. Chimäres Gen, umfassend in funktioneller Verknüpfung in Transkriptionsrichtung:
a) einen in einem pflanzlichen Plastiden funktionellen Promoter
b) eine Lumen-Targeting-Domäne einer Plastidentransitpeptidsequenz in translationeller Fusion mit
c) einer heterologen Nukleinsäuresequenz, die für ein Peptid codiert,
d) optional einen Terminator, der in den Plastiden von Pflanzenzellen aktiv ist,
**dadurch gekennzeichnet, dass** das chimäre Gen nicht die Stroma-Targeting-Domäne der Plastidentransitpeptidsequenz umfasst und wobei die Expression für das chimäre Gen mindestens 0,1% der gesamten löslichen Proteine ausmacht.

2. Chimäres Gen nach Anspruch 1, wobei die Nukleinsäuresequenz, die für ein Lumen-Targeting-Signalpeptid codiert, von einem vom Zellkern codierten Protein stammt, wobei für den Transport durch die Thylakoidmembran hindurch der Sec-abhängige Weg oder der Tat-Weg eingesetzt wird.

3. Chimäres Gen nach einem der Ansprüche 1 bis 2, wobei die heterologe Nukleinsäuresequenz, die für ein Peptid codiert, für das Aprotininprotein codiert.

4. Vektor für die Transformation von pflanzlichen Plastiden, **dadurch gekennzeichnet, dass** er mindestens zwei Sequenzen enthält, die zu Sequenzen im Plastom der zu transformierenden Pflanze homolog sind, wobei die homologen Sequenzen mindestens ein chimäres Gen nach einem der Ansprüche 1 bis 3 flankieren.

5. Transplastomische Pflanzenzelle, **dadurch gekennzeichnet, dass** sie mindestens ein chimäres Gen nach einem der Ansprüche 1 bis 3 enthält.

6. Transplastomische Pflanze und/oder transplastomischer Nachkomme davon, umfassend eine transplastomische Pflanzenzelle nach Anspruch 5.

7. Transplastomische Pflanze und/oder transplastomischer Nachkomme davon nach Anspruch 6, bei der/dem es sich um eine Alge, eine Lemnacee, eine Pflanze der Gattung *Nicotiana,* eine Kartoffel-, Tomaten- oder Sojabohnenpflanze handelt.

8. Erntbare Teile einer Pflanze nach einem der Ansprüche 6 bis 7, umfassend Pflanzenzellen nach Anspruch 5.

9. Verfahren zur Herstellung eines interessierenden Proteins in einer Pflanzenzelle, umfassend die folgenden Schritte:
a) Transformieren eines Plastiden mit einem chimären Gen nach einem der Ansprüche 1 bis 2;
b) Heranziehen einer Pflanzenzelle, umfassend den transformierten Plastiden, unter geeigneten Bedingungen für die Expression des chimären Gens und für die anschließende Abspaltung des Signalpeptids.

10. Verfahren zur Herstellung eines N-terminalen Peptids, bei dem es sich nicht um Methionin handelt, in einem pflanzlichen Plastiden, wobei das Verfahren Folgendes umfasst:
a) Transformieren eines Plastiden mit einem chimären Gen nach einem der Ansprüche 1 bis 2, wobei die heterologe Nukleinsäuresequenz für ein N-terminales Peptid, bei dem es sich nicht um Methionin handelt, codiert
b) Heranziehen einer Pflanzenzelle, umfassend den transformierten Plastiden, unter geeigneten Bedingungen für die Expression des chimären Gens und für die anschließende Abspaltung des Signalpeptids.

11. Verfahren nach Anspruch 10, wobei es sich bei dem N-terminalen Protein, bei dem es sich nicht um Methionin handelt, um Aprotinin handelt.

12. Verfahren zur Herstellung eines interessierenden Peptids, umfassend den Schritt, dass man das interessierende Peptid aus einer transplastomischen Pflanzenzelle nach Anspruch 5 oder aus einer transplastomischen Pflanze und/oder einem transplastomischen Nachkommen davon nach einem der Ansprüche 6 bis 7 oder aus erntbaren Teilen einer transplastomischen Pflanze nach Anspruch 8 extrahiert.

13. Verfahren nach Anspruch 12, wobei es sich bei dem interessierenden Peptid um Aprotinin handelt.

## Revendications

1. Gène chimère comprenant les éléments suivants liés les uns aux autres, de manière fonctionnelle, dans la direction de la transcription :
a) un promoteur fonctionnel dans un plaste de plante,
b) un domaine de ciblage du lumen d'une séquence de peptide de transit du plaste, fusionné de manière traductionnelle avec
c) une séquence d'acide nucléique hétérologue codant pour un peptide,
d) en option, un terminateur qui est actif dans les plastes des cellules végétales,
**caractérisé en ce que** ledit gène chimère ne comprend pas le domaine de ciblage du stroma provenant de ladite séquence de peptide de transit du plaste et où l'expression pour ledit gène chimère est d'au moins 0,1% des protéines solubles totales.

2. Gène chimère selon la revendication 1, dans lequel la séquence d'acide nucléique codant pour un peptide signal de ciblage du lumen provient d'une protéine codée dans le noyau, utilisant la voie dépendante de Sec ou la voie de Tat pour la translocation à travers la membrane thylacoïde.

3. Gène chimère selon l'une quelconque des revendications 1 à 2, dans lequel la séquence d'acide nucléique hétérologue codant pour un peptide code pour la protéine aprotinine.

4. Vecteur conçu pour la transformation de plastes de plantes, **caractérisé en ce qu'**il contient au moins deux séquences qui sont homologues à des séquences dans le plastome de la plante à transformer, lesdites séquences homologues flanquant au moins un gène chimère selon l'une quelconque des revendications 1 à 3.

5. Cellule végétale transplastomique **caractérisée en ce qu'**elle contient au moins un gène chimère selon l'une quelconque des revendications 1 à 3.

6. Plante transplastomique et/ou descendance de celle-ci comprenant une cellule végétale transplastomique selon la revendication 5.

7. Plante transplastomique et/ou descendance de celle-ci, selon la revendication 6, qui est une algue, une Lemnacée, une plante du genre *Nicotiana,* un plant de pomme de terre, de tomate ou de soja.

8. Parties récoltables d'une plante, selon l'une quelconque des revendications 6 à 7, comprenant des cellules végétales selon la revendication 5.

9. Procédé de production d'une protéine d'intérêt dans une cellule végétale, comprenant les étapes suivantes :
a) transformer un plaste avec un gène chimère selon l'une quelconque des revendications 1 à 2 ;
b) cultiver une cellule végétale comprenant ledit plaste transformé, dans des conditions appropriées pour l'expression du gène chimère et pour la coupure ultérieure du peptide signal.

10. Procédé de production d'un peptide, dont l'extrémité N-terminale n'est pas une méthionine, dans un plaste de plante, dans lequel ledit procédé comprend les étapes consistant à :
a) transformer un plaste avec un gène chimère, selon l'une quelconque des revendications 1 à 2, dans lequel la séquence d'acide nucléique hétérologue code pour un peptide dont l'extrémité N-terminale n'est pas une méthionine,
b) cultiver une cellule végétale comprenant ledit plaste transformé, dans des conditions appropriées pour l'expression du gène chimère et pour la coupure ultérieure du peptide signal.

11. Procédé selon la revendication 10, dans lequel la protéine, dont l'extrémité N-terminale n'est pas une méthionine, est l'aprotinine.

12. Procédé de production d'un peptide d'intérêt comprenant les étapes consistant à extraire le peptide d'intérêt à partir d'une cellule végétale transplastomique, selon la revendication 5, ou à partir d'une plante transplastomique et/ou sa descendance, selon l'une quelconque des revendications 6 à 7, ou à partir des parties récoltables d'une plante transplastomique selon la revendication 8.

13. Procédé selon la revendication 12, dans laquelle le peptide d'intérêt est l'aprotinine.
